# EUROPEAN PATENT APPLICATION

(11) **EP 3 489 242 A1**
(43) Date of publication of application: **29.05.2019**
(21) Application number: 17830497.8
(22) Date of filing: 21.07.2017
(51) Int. Cl.: C07D 519/00, A61P 31/06, A61P 31/00

(54) **CRYSTAL AND SALT OF NITROIMIDAZOLE, AND MANUFACTURING METHOD THEREOF**

(30) Priority: 22.07.2016 CN 201610587880
(71) Applicant: Medshine Discovery Inc., Nanjing, Jiangsu 310032 (CN)
(72) Inventor: LUO, Wei, Shanghai 200131 (CN); DING, Charles Z., Shanghai 200131 (CN); HUANG, Zhigang, Shanghai 200131 (CN); HU, Yinghu, Shanghai 200131 (CN); LI, Zongbin, Shanghai 200131 (CN); LU, Qingqing, Shanghai 200131 (CN)
(74) Representative: Schrell, Andreas
(86) International application number: PCT/CN2017/093809
(87) International publication number: WO 2018/014863

(57) **Abstract**

The present invention discloses a crystal form and salt of a nitroimidazole compound, and a manufacturing method thereof. The invention further comprises an application of the crystal form and salt in preparing a pharmaceutical product for preventing and treating an infection caused by *Mycobacterium tuberculosis* or another microbe.

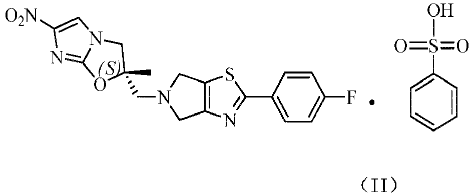

## Description

### Field of invention

The present invention relates to a crystal form and a salt of a nitroimidazole compound, and a preparation method thereof. The invention further comprises a use of the crystal form and the salt in manufacturing a medicament for preventing and treating an infection caused by *Mycobacterium tuberculosis* or another microbes.

### Background of invention

*Mycobacterium tuberculosis* is the pathogen of tuberculosis. Tuberculosis is a globally widespread and fatal infectious disease, more than 8 million people are infected with tuberculosis and 2 million people die from tuberculosis every year according to the World Health Organization. In the past decade, tuberculosis cases have grown at a rate of 20% worldwide, especially in poor areas. If this trend continues, tuberculosis cases are likely to continue to grow at a rate of 41% over the next two decades. In the 50 years since the initial application of chemotherapy, tuberculosis has been a main fatal infectious disease to adults, second only to AIDS.

The current treatment for tuberculosis is using a combination of multiple agentia recommended by the US Department of Public Health, including a first combined use of isoniazid, rifampicin, pyrazinamide and ethambutol for two months, followed by a combined use of Isoniazid and rifampin for four months. For patients infected with AIDS, the use of this combination of drugs needs to be extended to seven months. For patients infected with multidrug-resistant tuberculosis, an additional second-line agent, such as streptomycin, kanamycin, amikacin, capreomycin, ethionamide, cycloserine, ciprofloxacin and ofloxacin, also needs to be added into the pharmaceutical combination. Such medicaments for the combination therapies for patients with multidrug-resistant tuberculosis (usually over 2 years of treatment) usually have a lower activity and higher side effects compared to current first-line drugs on the market.

Currently, Otsuka's new product, Deltyba (delamanid), the compound code OPC-67683, has been approved for market used in adults and combination therapies for multidrug-resistant tuberculosis given the considerations for resistance and tolerance. The mechanism of action of Delamanid is to inhibit the synthesis of methoxy- and keto-mycolic acids which are important components of the cell wall of *Mycobacterium tuberculosis,* thereby killing bacteria. Delamanid has a high activity against multidrug-resistant tuberculosis both *in vitro* and *in vivo.* On November 21, 2013, Deltyba (Delamanid), a 50 mg thin-film coating tablet was conditionally approved by the European Committee for Medicinal Products for Human Use (CHMP) used for the treatment of multidrug-resistant tuberculosis. It was officially approved for market in Europe on April 28, 2014. Its structural formula is as follows:

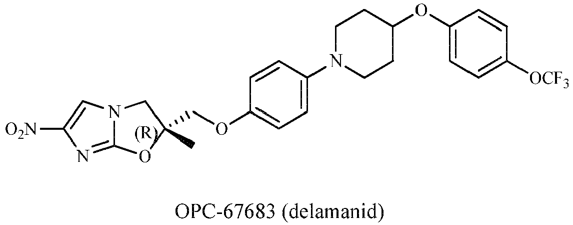

PCT/CN2016/072447 discloses a substituted nitroimidazole derivative which is mainly used for the treatment of related diseases caused by *mycobacterial* infection, such as *Mycobacterium tuberculosis,* especially for drug-resistant mycobacteria diseases. Its structure is as shown in formula (B-1):

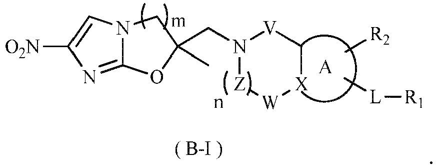

### Content of the present invention

The present invention provides a compound represented by formula (II)

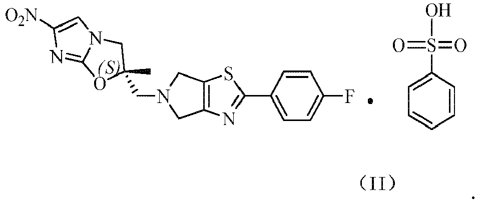

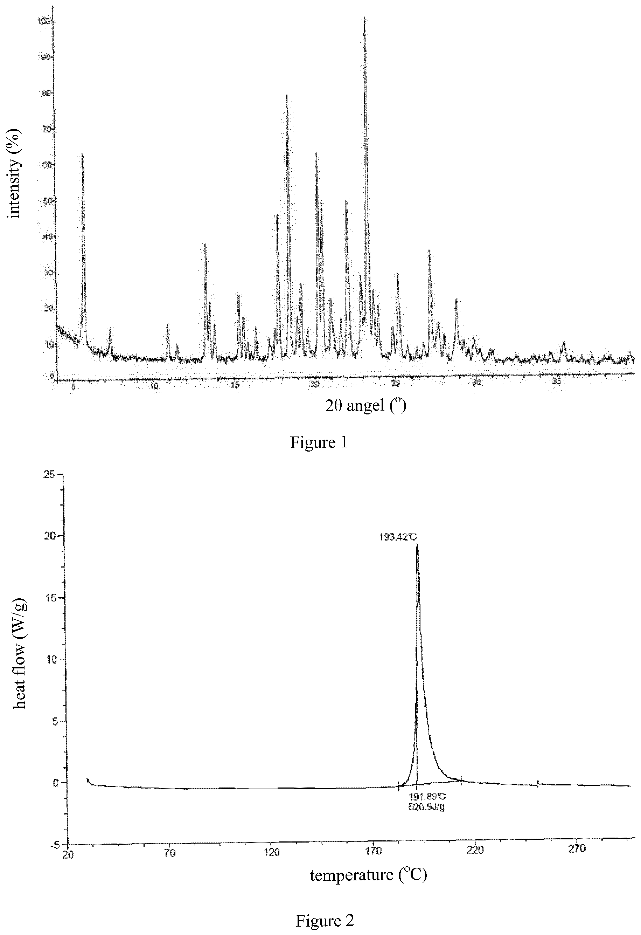

The present invention also provides a crystal form A of the compound represented by formula (II) whose X-ray powder diffraction pattern is as shown in Figure 1.

In certain embodiments of the present invention, the X-ray powder diffraction pattern analytic data of the crystal form A is as shown in Table 1,

**Table 1 X-ray powder diffraction pattern analytic data of the crystal form A of the compound represented by formula (II)**

| No. | 2θ angel (°) | interplanar spacing (Å) | relative intensity (%) | No. | 2θ angel (°) | interplanar spacing (Å) | relative intensity (%) |
|---|---|---|---|---|---|---|---|
| 1 | 5.736 | 15.3947 | 56.9 | 26 | 23.645 | 3.7596 | 18.5 |
| 2 | 7.335 | 12.042 | 8.1 | 27 | 23.962 | 3.7107 | 10.5 |
| 3 | 10.921 | 8.0943 | 10.5 | 28 | 24.884 | 3.5752 | 8.8 |
| 4 | 11.437 | 7.7306 | 5.1 | 29 | 25.204 | 3.5306 | 25.1 |
| 5 | 13.29 | 6.6563 | 34.4 | 30 | 25.757 | 3.4559 | 3.7 |
| 6 | 13.506 | 6.5504 | 17.3 | 31 | 26.357 | 3.3786 | 3.1 |
| 7 | 13.804 | 6.41 | 11.1 | 32 | 26.745 | 3.3305 | 3.9 |
| 8 | 15.304 | 5.7848 | 18.7 | 33 | 27.194 | 3.2766 | 31.7 |
| 9 | 15.594 | 5.6779 | 12.2 | 34 | 27.687 | 3.2192 | 10.3 |
| 10 | 15.819 | 5.5975 | 5 | 35 | 28.049 | 3.1786 | 6.2 |
| 11 | 16.352 | 5.4163 | 8.7 | 36 | 28.848 | 3.0923 | 17.2 |
| 12 | 17.193 | 5.1532 | 5.6 | 37 | 29.284 | 3.0472 | 5.2 |
| 13 | 17.554 | 5.0481 | 7.9 | 38 | 29.554 | 3.0201 | 1.8 |
| 14 | 17.787 | 4.9824 | 41.3 | 39 | 29.878 | 2.9881 | 6.3 |
| 15 | 18.477 | 4.798 | 76.9 | 40 | 30.247 | 2.9524 | 2.8 |
| 16 | 18.926 | 4.6851 | 11.3 | 41 | 30.897 | 2.8917 | 3.5 |
| 17 | 19.154 | 4.6299 | 21.2 | 42 | 31.041 | 2.8787 | 3 |
| 18 | 19.577 | 4.5307 | 7.3 | 43 | 32.503 | 2.7524 | 1.8 |
| 19 | 20.254 | 4.3809 | 58.6 | 44 | 34.598 | 2.5904 | 2.4 |
| 20 | 20.508 | 4.3271 | 45.2 | 45 | 35.495 | 2.527 | 5.5 |
| 21 | 21.038 | 4.2194 | 16.4 | 46 | 36.57 | 2.4551 | 1.9 |
| 22 | 21.65 | 4.1014 | 10.7 | 47 | 37.177 | 2.4164 | 2.3 |
| 23 | 22.068 | 4.0246 | 45.6 | 48 | 37.944 | 2.3693 | 1.8 |
| 24 | 22.894 | 3.8812 | 22.3 | 49 | 38.341 | 2.3457 | 2.1 |
| 25 | 23.329 | 3.8098 | 100 | 50 | 39.543 | 2.2771 | 3.1 |

In certain embodiments of the present invention, a crystal form A of the compound represented by formula (II), wherein, the X-ray powder diffraction pattern of the crystal form A has characteristic diffraction peaks at the following 2θ angles: 5.74±0.2°, 13.29±0.2°, 17.79±0.2°, 18.48±0.2°, 20.25±0.2°, 20.51±0.2°, 22.07±0.2°, 23.33±0.2°.

The present invention also provides a method for preparing the crystal form A, which comprises adding a compound represented by formula (I) in any form and benzenesulfonic acid to a solvent and crystallizing, 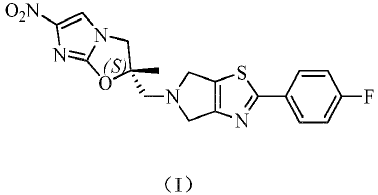 wherein,
the molar ratio of benzenesulfonic acid to the compound represented by formula (I) is from 1.2:1 to 1.0:1;
the solvent is used in an amount of 150 to 300 times the weight of the compound represented by formula (I);
the solvent is acetone.

In certain embodiments of the present invention, a method for preparing the crystal form A, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is used in an amount of 50 to 150 times the weight of the compound represented by formula (I);
the solvent is butanone.

In certain embodiments of the present invention, a method for preparing the crystal form A, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is used in an amount of 25 to 50 times the weight of the compound represented by formula (I);
the solvent is a mixed solvent of tetrahydrofuran and dimethyl sulfoxide.

In certain embodiments of the present invention, the method for preparing the crystal form A, wherein, the volume ratio of tetrahydrofuran to dimethyl sulfoxide is from 6:1 to 10:1.

In certain embodiments of the present invention, a method for preparing the crystal form A, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is used in an amount of 25 to 50 times the weight of the compound represented by formula (I);
the solvent is a mixed solvent of acetone and dimethyl sulfoxide.

In certain embodiments of the present invention, the method for preparing the crystal form A, wherein, the volume ratio of acetone to dimethyl sulfoxide is from 6:1 to 10:1.

In certain embodiments of the present invention, a method for preparing the crystal form A, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is used in an amount of 10 to 20 times the weight of the compound represented by formula (I);
the solvent is a mixed solvent of acetone and acetic acid.

In certain embodiments of the present invention, the method for preparing the crystal form A, wherein, the volume ratio of acetone to acetic acid is from 1:1 to 1.5:1.

The present invention also provides a crystal form B of the compound represented by formula (II), wherein, the X-ray powder diffraction pattern of the crystal form B has characteristic diffraction peaks at the following 2θ angles: 5.26±0.2°, 10.39±0.2°, 12.82±0.2°, 20.75±0.2°, 22.08±0.2°, 23.19±0.2°, 27.09±0.2°, 37.45±0.2°.

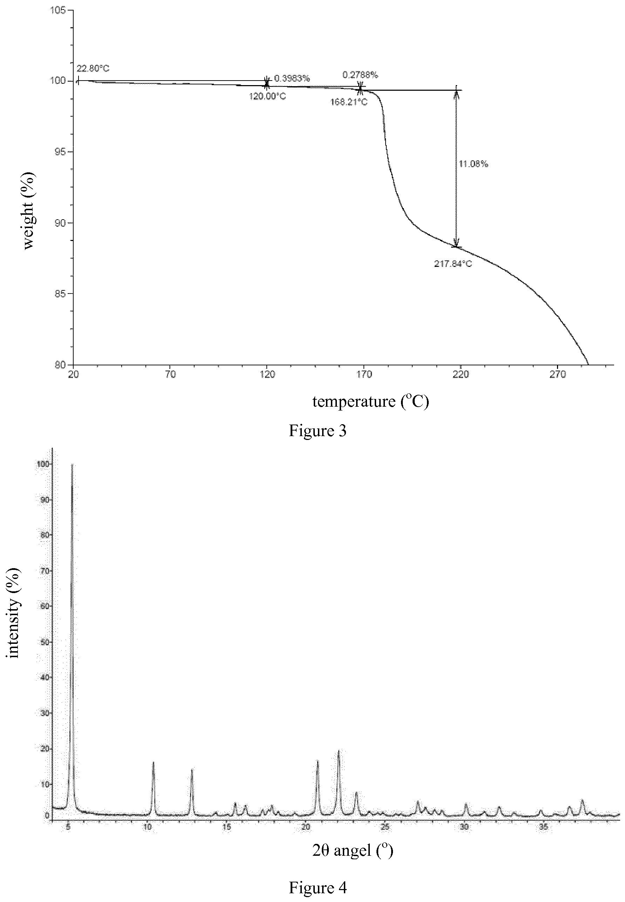

In certain embodiments of the present invention, the crystal form B of the compound represented by formula (II) whose X-ray powder diffraction pattern is as shown in Figure 4.

In certain embodiment of the present invention, the X-ray powder diffraction pattern analytic data of the crystal form B is as shown in Table 2,

**Table 2 X-ray powder diffraction pattern analytic data of the crystal form B of the compound represented by formula (II)**

| **No.** | **2θ angel (°)** | **interplanar spacing (Å)** | **relative intensity (%)** | **No.** | **2θ angel (°)** | **interplanar spacing (Å)** | **relative intensity (%)** |
|---|---|---|---|---|---|---|---|
| 1 | 5.261 | 16.7848 | 100 | 18 | 24.849 | 3.5801 | 0.9 |
| 2 | 10.39 | 8.5068 | 15.4 | 19 | 25.692 | 3.4645 | 0.6 |
| 3 | 12.816 | 6.9017 | 13.2 | 20 | 26.01 | 3.423 | 0.6 |
| 4 | 14.335 | 6.1738 | 1 | 21 | 27.093 | 3.2885 | 3.9 |
| 5 | 15.115 | 5.8568 | 0.5 | 22 | 27.568 | 3.2329 | 2.1 |
| 6 | 15.541 | 5.6972 | 3.6 | 23 | 28.139 | 3.1686 | 1.5 |
| 7 | 16.206 | 5.4648 | 2.8 | 24 | 28.575 | 3.1212 | 1.2 |
| 8 | 17.29 | 5.1246 | 1.5 | 25 | 30.112 | 2.9654 | 3.4 |
| 9 | 17.668 | 5.0158 | 1.7 | 26 | 31.276 | 2.8576 | 1.2 |
| 10 | 17.865 | 4.9608 | 2.9 | 27 | 32.223 | 2.7757 | 2.6 |
| 11 | 18.298 | 4.8446 | 1 | 28 | 33.111 | 2.7033 | 0.8 |
| 12 | 19.305 | 4.5941 | 0.9 | 29 | 34.792 | 2.5764 | 1.6 |
| 13 | 20.746 | 4.278 | 15.6 | 30 | 35.659 | 2.5157 | 0.7 |
| 14 | 22.085 | 4.0215 | 18.5 | 31 | 36.642 | 2.4505 | 2.5 |
| 15 | 23.192 | 3.8321 | 6.3 | 32 | 37.451 | 2.3994 | 4.4 |
| 16 | 23.999 | 3.705 | 1 | 33 | 37.94 | 2.3696 | 0.9 |
| 17 | 24.532 | 3.6257 | 0.8 | | | | |

The present invention also provides a method for preparing the crystal form B, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is used in an amount of 150 to 300 times the weight of the compound represented by formula (I);
the solvent is acetone.

The present invention also provides a compound represented by formula (III)

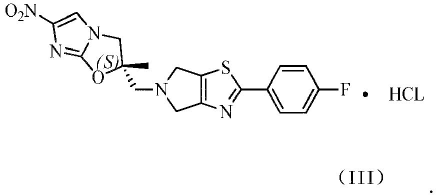

The present invention also provides a compound represented by formula (IV)

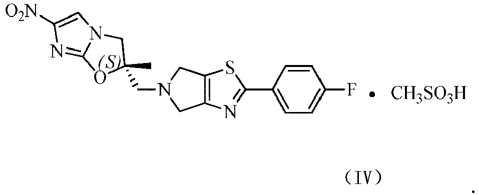

The present invention also provides a compound represented by formula (V)

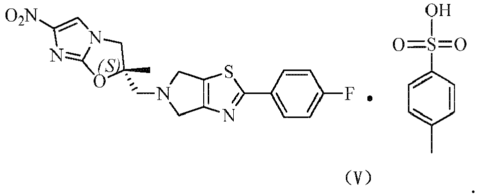

The present invention also provides a crystal form C of the compound represented by formula (I), wherein, the X-ray powder diffraction pattern of the crystal form C has characteristic diffraction peaks at the following 2θ angles: 7.18±0.2°, 10.78±0.2°, 14.10±0.2°, 14.41±0.2°, 15.36±0.2°, 23.72±0.2°, 25.36±0.2°, 27.49±0.2°.

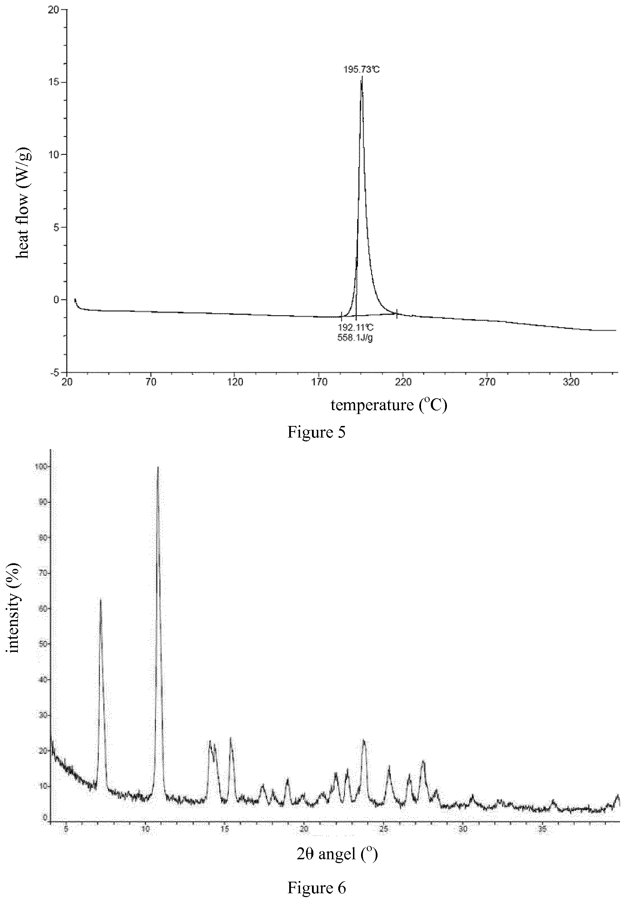

In certain embodiments of the present invention, the crystal form C of the compound represented by formula (I) whose X-ray powder diffraction pattern is as shown in Figure 6.

In certain embodiments of the present invention, the X-ray powder diffraction pattern analytic data of the crystal form C is as shown in Table 3,

**Table 3 X-ray powder diffraction pattern analytic data of the crystal form C of the compound represented by formula (I)**

| No | 2θ angel (°) | Interplanar spacing (Å) | relative intensity (%) | No | 2θ angel (°) | Interplanar spacing (Å) | relative intensity (%) |
|---|---|---|---|---|---|---|---|
| 1 | 7.177 | 12.3072 | 57 | 13 | 22.717 | 3.9112 | 9.7 |
| 2 | 10.784 | 8.1968 | 100 | 14 | 23.723 | 3.7474 | 18.6 |
| 3 | 14.097 | 6.2774 | 18.5 | 15 | 25.358 | 3.5095 | 11.5 |
| 4 | 14.413 | 6.1403 | 16 | 16 | 26.62 | 3.3459 | 8.4 |
| 5 | 15.363 | 5.7628 | 19.1 | 17 | 27.49 | 3.2419 | 11.7 |
| 6 | 17.429 | 5.0841 | 5.6 | 18 | 28.337 | 3.1469 | 3.8 |
| 7 | 18.021 | 4.9183 | 4.1 | 19 | 30.589 | 2.9202 | 3.8 |
| 8 | 18.988 | 4.6699 | 7.6 | 20 | 32.386 | 2.7621 | 2.3 |
| 9 | 19.912 | 4.4554 | 2.9 | 21 | 35.694 | 2.5133 | 3.5 |
| 10 | 21.081 | 4.2108 | 2.8 | 22 | 39.132 | 2.3001 | 1.5 |
| 11 | 21.711 | 4.09 | 4.8 | 23 | 39.758 | 2.2653 | 3.8 |
| 12 | 22.026 | 4.0322 | 8.7 | | | | |

The present invention also provides a use of the compound, the crystal form A, the crystal form B or the crystal form C in manufacturing a medicament for preventing and treating an infection of *Mycobacterium tuberculosis* or other microbes.

### Technical effect

The crystal form A, the crystal form B of the compound represented by formula (II) and the crystal form C of the compound represented by formula (I) provided by the present invention have a stable property, a good solubility and a good wettability, and have a good pharmaceutical prospect.

The crystal form A, the crystal form B of the compound represented by formula (II) and the crystal form C of the compound represented by formula (I) provided by the present invention are easy to be prepared, and the reagents used are common, readily available on the market and inexpensive; the solvents are environmentally friendly, and most belong to the class 3 solvents; a single stable crystal form can be obtained by a variety of solvents; simple operation, mild conditions; good purity, high yield.

### Definitions and descriptions

Unless otherwise stated, the following terms and phrases as used herein are intended to have the following meanings. A particular phrase or term should not be considered undefined or unclear without a particular definition, but should be understood in the ordinary sense. When a trade name appears herein, it is intended to refer to its corresponding commodity or its active ingredient.

The intermediate compounds in the present invention can be prepared by a variety of synthetic methods well-known to those skilled in the art, including the specific embodiments listed below, combinations thereof with other chemical synthesis methods, and equivalents well-known to those skilled in the art. Preferred embodiments include, but are not limited to, embodiments in the present invention.

The chemical reaction of a specific embodiment in the present invention is carried out in a suitable solvent which is suitable for the chemical changes of the present invention and the reagents and materials required thereof. In order to obtain the compound of the present invention, it is sometimes necessary for those skilled in the art to modify or select synthetic steps or reaction schemes based on the existing embodiments.

The present invention is specifically described by the following embodiments, which are not intended to limit the present invention.

All solvents used in the present invention are commercially available and can be used without further purification.

The present invention employs the following abbreviations: aq. is water; equivalent is equivalent; THF is tetrahydrofuran; DMSO is dimethyl sulfoxide; AcOH is acetic acid; TsOH is *p*-toluenesulfonic acid; CH₃SO₃H is methanesulfonic acid; H₂SO₄ is sulfuric acid; HCl is hydrochloric acid; CuI stands for cuprous iodide; CuBr stands for cuprous bromide; CuCl stands for cuprous chloride; Cu stands for copper powder; Cu₂O stands for cuprous oxide; DMF stands for *N,N-*dimethylformamide; TFA stands for trifluoroacetic acid.

Compounds are named manually or by ChemDraw® software, and commercial compounds are listed under the supplier's catalogue.

Method for X-ray powder diffractometer (XRPD) in the present invention
Instrument model: Bruker D8 advance X-ray diffractometer
Testing method: about 10 - 20 mg sample was used for XRPD.

Detailed XRPD parameters were as follows:
Light pipe: Cu, kα, (λ=1.54056Ǻ)
Light pipe voltage: 40 kV, light pipe current: 40 mA
Divergence slit: 0.60 mm
Detector slit: 10.50 mm
Anti-scattering slit: 7.10 mm
Scan range: 4-40 deg
Step diameter: 0.02 deg
Step length: 0.12 s
Sample disc speed: 15 rpm.

Method for Differential Scanning Calorimeter (DSC) in the present invention
Instrument model: TA Q2000 differential scanning calorimeter
Testing method: the sample (about 1 mg) was tested in a DSC aluminum pan. The sample was heated from 25°C to 300°C (or 350°C) at a heating rate of 10°C/minute under 50 mL/minute N₂.

Method for Thermal Gravimetric Analyzer (TGA) in the present invention
Instrument model: TA Q5000IR thermal gravimetric analyzer
Testing method: the sample (2 to 5 mg) was tested in a TGA aluminum pan. The sample was heated to lose 20% weight from room temperature at a heating rate of 10°C/minute under 25 mL/minute N₂.

### Brief description of the drawings

Fig.1 is Cu-Kα radiation XRPD pattern of the crystal form A of the compound represented by formula (II).
Fig.2 is DSC spectrum of the crystal form A of the compound represented by formula (II).
Fig.3 is TGA spectrum of the crystal form A of the compound represented by formula (II).
Fig.4 is Cu-Kα radiation XRPD pattern of the crystal form B of the compound represented by formula (II).
Fig.5 is DSC spectrum of the crystal form B of the compound represented by formula (II).
Fig.6 is Cu-Kα radiation XRPD pattern of the crystal form C of the compound represented by formula (I).

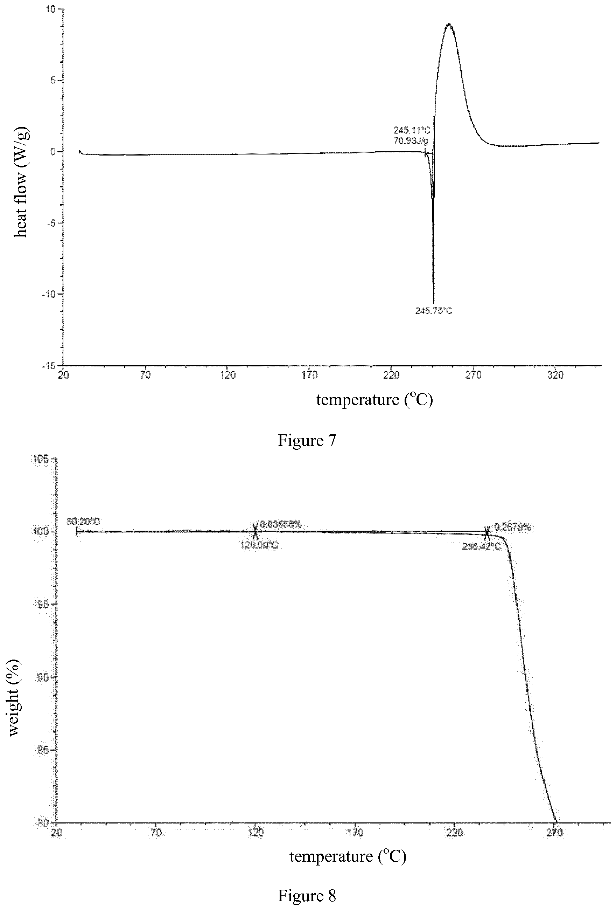

Fig.7 is DSC spectrum of the crystal form C of the compound represented by formula (I).
Fig.8 is TGA spectrum of the crystal form C of the compound represented by formula (I).

### Detailed description of the preferred embodiment

In order to better understand the content of the present invention, the following further describes the specific embodiments, but the specific embodiments are not limited to the contents of the present invention.

### Embodiment 1

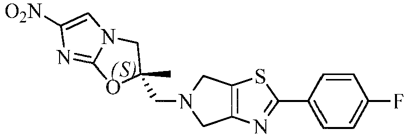

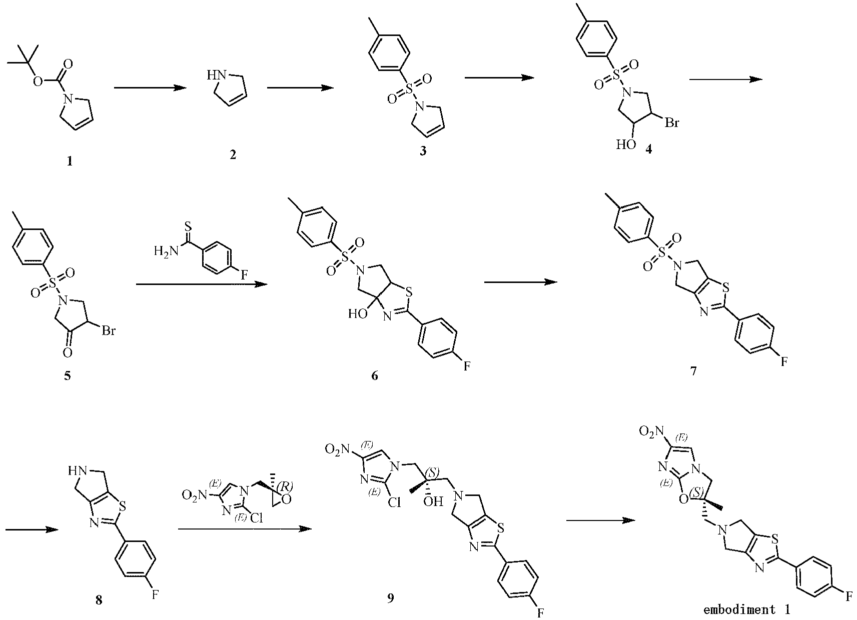

### Step 1:

To a solution of *tert*-butyl 2,5-dihydropyrrole-1-carboxylate (2.00 g, 11.82 mmol, 1.00 equivalent) in dichloromethane (6.00 mL) was added TFA (9.18 g, 80.51 mmol, 6.81 equivalent) and the mixture was stirred at 20°C for 1 hour. The mixture was concentrated under reduced pressure to give a crude product as a black-brown oil which was directly used for the next step.

### Step 2:

To a solution of 2,5-dihydro-1*H*-pyrrole (2.16 g, 11.79 mmol, 1.00 equivalent, TFA) in dichloromethane (10.00 mL) was added TosCl (2.70 g, 14.15 mmol, 1.20 equivalent) and triethylamine (3.58 g, 35.37 mmol, 3.00 equivalent), and the mixture was stirred at 20°C for 16 hours. The mixture was washed with 1M HCl (30 mL), saturated aqueous NaHCO₃ (30 mL) and aqueous NaCl (30 mL). The organic phase was dried over anhydrous Na₂SO₄, filtered and the filtrate was concentrated under reduced pressure to give 1-tosyl-2,5-dihydro-pyrrole (1.50 g, 6.72 mmol, 56.98% yield) as a brown solid.

### Step 3:

To a solution of 1-tosyl-2,5-dihydropyrrole (1.50 g, 6.72 mmol, 1.00 equivalent) in DMSO (10.00 mL) and acetonitrile (5.00 mL) was added NBS (1.79 g, 10.08 mmol, 1.50 equivalent) in batches at 0°C. Then the mixture was stirred at 15°C for 16 hours. The mixture was diluted with water (50 mL) and extracted with ethyl acetate (30 mL×2). The combined organic phases were washed with aqueous NaCl (30 mL), dried over anhydrous Na₂SO₄, filtered and the filtrate was concentrated under reduced pressure. The residue was purified by silica gel column chromatography (silicon dioxide, PE/EA=10:1 to 3:1) to give 4-bromo-1-tosylpyrrolidin-3-ol (1.58 g, 4.93 mmol, 73.43% yield) as a white solid.

### Step 4:

To a solution of 4-bromo-1-tosylpyrrolidin-3-ol (1.58 g, 4.93 mmol, 1.00 equivalent) in dichloromethane (15.00 mL) was added Dess-Martin reagent (4.19 g, 9.87 mmol, 2.00 equivalent) and the mixture was purged with N₂. The mixture was stirred at 10-15°C for 12 hours. The mixture was washed with aqueous NaHCO₃ (50 mL×2) and extracted with ethyl acetate (30 mL×2). The combined organic layers were dried over anhydrous Na₂SO₄, filtered and the filtrate was concentrated under reduced pressure. The residue was purified by silica gel column chromatography (silicon dioxide, PE/EA= 10:1 to 1:1) to give 4-bromo-1-tosylpyrrolidin-3-one (980.00 mg, 3.08 mmol, 62.47% yield) as a yellow solid. ¹H NMR (300MHz, CDCl₃) 7.69-7.61 (d*,J* = 8.1 Hz, 2H), 7.35-7.28 (d*,J* = 7.8 Hz, 2H), 4.32-4.25 (t, *J* = 6.3 Hz, 1H), 4.00-3.91 (m, 1H), 3.61 (s, 2H), 3.55-3.47 (m, 1H), 2.39 (s, 3H).

### Step 5:

To a solution of 4-bromo-1-tosylpyrrolidin-3-one (880.00 mg, 2.77 mmol, 1.00 equivalent) in DMF (12.00 mL) was added 4-fluorobenzothioamide (430.00 mg, 2.77 mmol, 1.00 equivalent) and the mixture was stirred at 60°C for 16 hours. The mixture was concentrated under reduced pressure, and the residue was diluted with ethyl acetate (10 mL) and filtered. The filter cake was collected and the filtrate was purified by silica gel column chromatography (silicon dioxide, PE/EA=10:1 to 3:1) to give 2-(4-fluorophenyl)-5-tosyl-4,5,6,6a-tetrahydro-3a*H*-pyrrolo[3,4-*d*]thiazol-3a-ol (900.00 mg, 2.49 mmol) as a gray solid.

### Step 6:

2-(4-Fluorophenyl)-5-tosyl-4,5,6,6a-tetrahydro-3a*H*-pyrrolo[3,4-*d*]thiazol-3a -ol (900.00 mg, 2.49 mmol, 1.00 equivalent), methanesulfonyl chloride (444.00 mg, 3.88 mmol, 1.69 equivalent) and triethylamine (730.00 mg, 7.21 mmol, 3.15 equivalent) were dissolved in dichloromethane (20.00 mL), and the mixture was degassed and purged with N₂. Then the mixture was stirred at 20°C for 16 hours, diluted with water (50 mL) and extracted with dichloromethane (30 mL×2). The combined organic phases were dried over anhydrous Na₂SO₄, filtered and the filtrate was concentrated under reduced pressure. The residue was purified by silica gel column chromatography (silicon dioxide), PE/EA= 20:1 to 1:1) to give 2-(4-fluorophenyl)-5-tosyl-5,6-dihydro-4*H*-pyrrolo[3,4-*d*]thiazole (600.00 mg, 1.11 mmol, 48.28% yield, 69% purity) as a light yellow solid.

### Step 7:

To a solution of 2-(4-fluorophenyl)-5-tosyl-5,6-dihydro-4*H*-pyrrolo[3,4-*d*]thiazole (550.00 mg, 1.01 mmol, 1.00 equivalent) in water (3.00 mL) and AcOH (15.00 mL) was added a solution of hydrogen bromide in acetic acid (81.72 mg, 1.01 mmol, 1.00 equivalent). The mixture was stirred at 20°C for 1 hour. The mixture was concentrated under reduced pressure, diluted with water (50 mL) and extracted with ethyl acetate (50 mL). The pH of the aqueous phase was adjusted to 11 with NaOH, and the aqueous phase was extracted with ethyl acetate (40 mL×2). The organic phase was dried over anhydrous Na₂SO₄, filtered and the filtrate was concentrated under reduced pressure to give 2-(4-fluorophenyl)-5,6-dihydro-4*H*-pyrrolo[3,4-*d*]thiazole (145.00 mg, 658.28 µmol, 65.18% yield) as a light yellow solid.

### Step 8:

To a solution of 2-(4-fluorophenyl)-5,6-dihydro-4*H*-pyrrolo[3,4-*d*]thiazole (160.00 mg, 726.38 µmol, 1.00 equivalent) was added 2-chloro-1-[[(2*R*)-2-methyloxiran-2-yl]methyl]-4-nitroimidazole (158.00 mg, 726.07 µmol, 1.00 equivalent) and diisopropylamine (481.00 mg, 3.72 mmol, 5.12 equivalent). The mixture was stirred at 80°C for 16 hours, diluted with water (50 mL) and extracted with ethyl acetate (30 mL×2). The combined organic phases were dried over anhydrous Na₂SO₄, filtered and the filtrate was concentrated under reduced pressure. The residue was purified by silica gel column chromatography (silicon dioxide, dichloromethane/methanol=20:1) to give (25)-1-(2-chloro-4-nitroimidazol-1-yl)-3-(2-(4-fluorophenyl)-4,6-dihydropyrrolo[3,4-*d*]thiazol-5-yl)-2-methylpropan-2-ol (150.00 mg, 342.56 µmol, 47.16% yield) as a light yellow solid.

### Step 9:

To a solution of (2*S*)-1-(2-chloro-4-nitroimidazol-1-yl)-3-(2-(4-fluorophenyl)-4,6-dihydropyrrolo[3,4-*d*]thiazol-5-yl)-2-methylpropan-2-ol (150.00 mg, 342.56 µmol, 1.00 equivalent) in DMF (5.00 mL) was added NaH (30.00 mg, 750 µmol, 2.19 equivalent) under N₂ atmosphere at 0°C and the mixture was stirred for 1 hour. The mixture was poured into saturated aqueous NH₄Cl (40 mL) and extracted with ethyl acetate (30 mL×2). The combined organic phases were dried over anhydrous Na₂SO₄, filtered and the filtrate was concentrated under reduced pressure. The residue was purified by ethyl acetate to give embodiment 1 (17.71 mg, 40.90 µmol, 11.94% yield, 92.71% purity). ¹H NMR (400MHz, CDCl₃) 7.91-7.84 (m, 2H), 7.57 (s, 1H), 7.17-7.09 (t, *J* = 8.4 Hz, 2H), 4.53-4.48 (d, *J* = 9.6 Hz, 1H), 4.20 (d, *J* = 3.3 Hz, 3H), 4.13-4.07 (m, 1H), 4.03-3.97 (m, *J* = 9.6 Hz, 1H), 3.42 (d, *J* = 14.8 Hz, 1H), 3.12 (d, *J* = 14.8 Hz, 1H), 1.64-1.60 (m, 3H). LCMS (ESI) *m*/*z*: 402.10 (M+1).

### Embodiment 2 the preparation of the crystal form A of the compound represented by formula (II)

The compound represented by formula (I) (500 mg, 1.25 mmol) was added into acetone (75 mL), and the mixture was warmed to 50°C and stirred to be clear, and then benzenesulfonic acid (593 mg, 3.75 mmol) was added. The mixture was stirred at 50°C for 10 minutes. Lots of solid precipitated, and the mixture was cooled to room temperature slowly and filtered. The filter cake was washed with few acetone and dried to give the product as a grey solid (500 mg, 0.89 mmol), 97.4% purity of HPLC and 71.5% yield.

### Embodiment 3 the preparation of the crystal form A of the compound represented by formula (II)

The compound represented by formula (I) (4.8 g, 11.96 mmol) was added into a mixed solution of DMSO (17 mL) and acetone (140 mL), and the mixture was warmed to 60°C, and then benzenesulfonic acid (2.08 g, 13.16 mmol) dissolved in acetone (30 mL) was added into the reaction flask dropwise. Lots of solid precipitated, and the mixture was cooled to 40°C and stirred for 1 hour. The mixture was cooled to 15°C and stirred for 11 hours, then filtered. The filter cake was washed with few acetone and dried to give the product as an off-white solid, which was dissolved in acetone (40 mL). The mixture was stirred at 40°C for 12 hours, and filtered to give the product as an off-white solid (5.20 g, 9.22 mmol), 99.2% purity of HPLC and 77.0% yield.

### Embodiment 4 the preparation of the crystal form A of the compound represented by formula (II)

The compound represented by formula (I) (100 mg, 0.249 mmol) was added into a mixed solution of DMSO (0.35 mL) and butanone (3.5 mL), and the mixture was warmed to 60°C and stirred to be clear, and then benzenesulfonic acid (43.3 mg, 0.274 mmol) dissolved in butanone (0.1 mL) was added into the reaction flask dropwise. Lots of solid precipitated, and the mixture was cooled to 40°C and stirred for 1 hour. The mixture was cooled to 15°C and stirred for 11 hours, then filtered. The filter cake was washed with few acetone and dried to give the product as an off-white solid (110 mg, 0.195 mmol), 99.4% purity of HPLC and 78.4% yield.

### Embodiment 5 the preparation of the crystal form A of the compound represented by formula (II)

The compound represented by formula (I) (1.00 g, 2.49 mmol) was added into a mixed solution of acetone (10 mL) and acetic acid (10 mL), and the mixture was stirred evenly at room temperature of 15°C and became a suspension. Benzenesulfonic acid (433 mg, 2.74 mmol) was added to the mixture, then the solid in the suspension was dissolved and the mixture became clear. The mixture was stirred for 12 hours overnight. Lots of solid precipitated, and was filtered. The filter cake was washed with few acetone and dried to give the product as an off-white solid (1.25 g, 2.22 mmol), 99.2% purity of HPLC and 88.9% yield.

### Embodiment 6 the preparation of the crystal form B of the compound represented by formula (II)

The compound represented by formula (I) (6.00 g, 14.95 mmol) was added into acetone (600 mL), and the mixture was warmed to 60°C and stirred to be clear, and then benzenesulfonic acid (2.60 g, 16.45 mmol) dissolved in acetone (30 mL) was added into the reaction flask dropwise. Lots of solid precipitated. The mixture was cooled to room temperature and stirred for 12 hours, then filtered. The filter cake was washed with few acetone and dried to give the product as a white solid (7.10 g, 12.64 mmol), 99.6% purity of HPLC and 84.5% yield.

### Embodiment 7 the preparation of the crystal form C of the compound represented by formula (I)

The compound represented by formula (V) (3.89 g, 6.78 mmol) was mixed with THF (100 mL) and solid NaHCO₃ (854.57 mg, 10.17 mmol) was added to the mixture. The mixture was stirred at 25°C for 15 minutes. The mixture was concentrated and washed with water (200 mL). The aqueous solution was extracted with dichloromethane (300 mL x 3) and the extract was washed with saturated aqueous NaCl. The organic phase was concentrated. The residue was pulped with methanol (50 mL) to give the product as a white solid (2.51 g, 6.22 mmol), 99.4% purity of HPLC and 91.7% yield.

### Embodiment 8 the preparation of the compound represented by formula (III)

The compound represented by formula (I) (80 mg, 0.199mmol) was dissolved in THF (2 mL) and DMSO (200 µL) and the mixture was heated to 50°C to be clear. A mixed solvent of hydrochloric acid (20 µL, 0.24 mmol) and THF (50 µL) was added to the mixture and the resulting mixture was stirred at 50°C for 2 hours. The mixture was cooled to room temperature and stirred overnight. The mixture was centrifuged rapidly, the precipitate was collected and dried to give the product as a white solid.

### Embodiment 9 the preparation of the compound represented by formula (IV)

The compound represented by formula (I) (80 mg, 0.199mmol) was dissolved in THF (2 mL) and DMSO (200 µL), the mixture was heated to 50°C to be clear. A mixed solvent of methanesulfonic acid (15 µL, 0.234 mmol) and THF (50 µL) was added to the mixture and the resulting mixture was stirred at 50°C for 2 hours. The mixture was cooled to room temperature and stirred overnight. The mixture was centrifuged rapidly, the precipitate was collected and dried to give the product as a white solid.

### Embodiment 10 the preparation of the compound represented by formula (V)

The compound represented by formula (I) (80 mg, 0.199mmol) was dissolved in THF (2 mL) and DMSO (200 µL), the mixture was heated to 50°C to be clear. A mixed solvent of *p*-toluenesulfonic acid (42.26 mg, 0.245 mmol) and THF (50 µL) was added to the mixture and the resulting mixture was stirred at 50°C for 2 hours. The mixture was cooled to room temperature and stirred overnight, and few solid precipitated. Ethyl acetate was added to the mixture as an anti-solvent and lots of solid precipitated. The mixture was centrifuged rapidly, the precipitate was collected and dried to give the product as a white solid.

### Stability test in different solvents for the crystal form A of the compound represented by formula (II)

30 mg of the crystal form A of the compound represented by formula (II) was taken in multiple portions, and 0.2 mL of a single or mixed solvent in the table below was added, respectively. The mixture was stirred at 40°C for 2 days, and then centrifuged. The solid in all samples was collected, dried in a vacuum oven (40°C) overnight, and was tested for crystal form by XRPD. The results were as shown in Table 4.

**Table 4 Stability test in different solvents for the crystal form A of the compound represented by formula (II)**

| Number | Solvent | Appearance (2 days) | Result |
|---|---|---|---|
| 1 | ethanol | suspension | crystal form A |
| 2 | isopropanol | suspension | crystal form A |
| 3 | acetonitrile | suspension | crystal form A |
| 4 | ethyl acetate | suspension | crystal form A |
| 5 | ethanol-water (1:1) | suspension | crystal form A |
| 6 | isopropanol-water (1:1) | suspension | crystal form A |
| 7 | acetonitrile-water (1:1) | suspension | crystal form A |

### Solid stability test under high temperature and high humidity conditions for the crystal form A of the compound represented by formula (II)

According to the "Guidelines for the Stability Test of APIs and Preparations" (Chinese Pharmacopoeia 2015 edition Part IV general rules 9001), stability of the crystal form A of the compound represented by formula (II) was tested at high temperature (60°C, open), high humidity (room temperature/relative humidity 92.5%, open).

Approximately 7 mg of the sample of the crystal form A of the compound represented by formula (II) was weighed and placed at the bottom of a glass vial to form a thin layer. Samples placed under high temperature and high humidity conditions were sealed with aluminum foil paper and small holes were punched in the aluminum foil paper to ensure that the sample could fully contact with ambient air; samples placed under strong light conditions were sealed with threaded caps. The samples placed under different conditions were sampled and tested on the 5th day and 10th day, and the test results were compared with the initial test results of 0 days. The test results were as shown in Table 5 below:

**Table 5 Solid stability test for the crystal form A of the compound represented by formula (II)**

| **Test conditions** | **Time point** | **Appearance** | **XRPD** | **Total impurity (%)** |
|---|---|---|---|---|
| - | 0 day | grey powder | crystal form A | 1.32 |
| high temperature (60°C, open) | 5th day | grey powder | crystal form A | 1.49 |
| | 10th day | | crystal form A | 1.86 |
| high humidity (room temperature/relative humidity 92.5%, open) | 5th day | grey powder | crystal form A | 1.32 |
| | 10th day | | crystal form A | 1.60 |

### Solubility test in different solvents for the crystal form C of the compound represented by formula (I)

The solubility test was carried out by using a manual stepwise dilution method under normal temperature conditions while observing the dissolution. About 2 mg of the crystal form C of the compound represented by formula (I) was added into different vials used in the liquid phase, and then an organic solvent or solvent mixture was added in small portions to observe the dissolution of the compound.

The solubility test results were as shown in Table 6.

**Table 6 Solubility test in different solvents for the crystal form C of the compound represented by formula (I)**

| **Number** | **Solvent** | **Solubility (mg/mL)** |
|---|---|---|
| 1 | methanol | <2 |
| 2 | ethanol | <2 |
| 3 | isopropanol | <2 |
| 4 | *n*-butanol | <2 |
| 5 | acetonitrile | 5-10 |
| 6 | acetone | 5-10 |
| 7 | methyl ethyl ketone | 5-10 |
| 8 | methyl isopropyl ketone | <2 |
| 9 | ethyl acetate | <2 |
| 10 | isopropyl acetate | <2 |
| 11 | *tert*-butyl methyl ether | <2 |
| 12 | tetrahydrofuran | 5-10 |
| 13 | 2 -methyltetrahydro furan | <2 |
| 14 | toluene | <2 |
| 15 | heptane | <2 |
| 16 | cyclohexane | <2 |
| 17 | 1,4-dioxane | <2 |
| 18 | water | <2 |
| 19 | methanol-water(1:1) | <2 |
| 20 | methanol-water (3:1) | <2 |
| 21 | ethanol-water (1:1) | <2 |
| 22 | ethanol-water (3:1) | <2 |
| 23 | acetonitrile-water(1:1) | <2 |
| 24 | acetone-water(1:2) | <2 |
| 25 | isopropanol-water(1:1) | <2 |

In vitro efficacy assay of the compound represented by formula (I)

### Assay method:

The compound represented by formula (I) was dissolved in pure DMSO (Sigma 276855-2L) to a concentration of 10 mg/ml as the mother liquor of the compound. 30 µL DMSO was added to the wells of the 2^{nd} to 11^{th} columns of a v-bottom 96-well plate (Axygen-wipp02280). 30 µL of the mother liquor of the compound was added to the 2^{nd} column wells and mixed. 30 µL of the liquid in the 2^{nd} column wells was transferred to the 3^{rd} column wells and mixed by pipette. Such operations were repeated until the 10^{th} column. The 11^{th} column wells were not added with the compound and contained only 30 µL DMSO. This plate was the "mother-plate" of the compound. From the 2^{nd} column to 11^{th} column, the corresponding compound concentrations were 5, 2.5, 1.25, 0.625, 0.3125, 0.156, 0.078, 0.039, 0.02, 0 mg/ml respectively. For the compound with a relatively good efficacy, the test concentration was reduced appropriately. A flat-bottom 96-well plate (Greiner 655090) was used as the "sub-plate". 98 µL of 7H9 medium (Sigma M0178) was added to all wells of the sub-plate. 2 µL compound was transferred from the mother-plate into the sub-plate at the corresponding position. The row A and row H, 1^{st} column and 12^{th} column of the sub-plate contained only 7H9 medium.

The H37Rv strain in a glycerol cryogenic vial was inoculated into 7H9 medium containing 0.05% Tween 80, and cultured in a shaker at 200 rpm/minute at 37°C for 4 weeks. The bacterial solution was washed twice with 7H9 medium containing 0.05% Tween 80 and resuspended in the same medium. The absorbance of the bacterial solution, OD₅₅₀, was adjusted to between 0.4 and 0.5 with the same medium. This bacterial solution was dispensed into a microcentrifuge tube and stored at -80°C. Storage time was less than 1 month. On the onset day of the test, the dispensed bacteria were thawed. The thawed bacterial solution was diluted 20-fold with 7H9 medium and then diluted 50-fold (a total of 1000 times). This bacterial solution was used to inoculate the sub-plate. 100 µL of the bacterial solution was inoculated into each well of the sub-plate, and the 12^{th} column wells were only added with 100 µL 7H9 medium with no bacterial solution added.

The test sub-plate was placed in a 37°C incubator and the humidity was maintained at >80%. After one week, 12.5 µL 7H9 medium containing 20% Tween 80 and 20 µL Alamar Blue (Invitrogen DAL1100) were added to the 1^{st} column wells containing bacteria and the 12^{th} column wells containing no bacteria every day. The sub-plate was inoculated for 24 hours and observed. When the bacterial solution in the 1^{st} column wells could reduce the added Alamar Blue to pink within 24 hours, 7H9 medium containing 20% Tween 80 and Alamar Blue were added to all wells of the test plate and the plate was incubated at 37°C for 24 hours, and then the fluorescence values were measured.

The compound represented by formula (I) was dissolved in pure DMSO (Sigma 276855-2L) to a concentration of 12.8 mg/mL as the mother liquor of the compound. 30 µL DMSO was added to the wells of the 1^{st} to 12^{th} columns of a v-bottom 96-well plate (Axygen-wipp02280). 30 µL of the mother liquor of the compound was added to the 1^{st} column. 30 µL of the 1^{st} column wells was transferred into the 2^{nd} column wells and mixed by pipette. Such operations for 2-fold gradient dilution were repeated until 11^{th} column. 12^{th} column was not added with the compound and contained only 30 µL DMSO. Only 30 µL DMSO was added to each of row A wells and row H wells. This plate was the "mother-plate" of the compound. From 1^{st} column to 12^{th} column, the corresponding compound concentrations were 6.4, 3.2, 1.6, 0.8, 0.4, 0.2, 0.1, 0.05, 0.025, 0.0125, 0.00625 and 0 mg/ml respectively.. For compounds with a relatively good efficacy, the test concentration was reduced appropriately. A flat-bottom 96-well plate (Greiner 655090) was used as the "sub-plate". 98 µL of 7H9 medium (Sigma M0178) was added to all wells of the sub-plate. 2 µL compound was transferred from the mother-plate into the sub-plate at the corresponding position. The row A and row H, 12^{th} column of the sub-plate contained only 7H9 medium and DMSO at a corresponding concentration.

The BCG strain in a glycerol cryogenic vial was inoculated into 7H9 medium containing 0.05% Tween 80, and cultured in a shaker at 200 rpm/minute at 37°C for 4 weeks. The bacterial solution was washed twice with 7H9 medium containing 0.05% Tween 80 and resuspended in the same medium. The absorbance of the bacterial solution, OD₅₅₀, was adjusted to between 0.4 and 0.5 with the same medium. This bacterial solution was dispensed into a microcentrifuge tube and stored at -80°C. Storage time was less than 1 month. On the onset day of the test, the dispensed bacteria were thawed. The thawed bacterial solution was diluted 20-fold with 7H9 medium and then diluted 50-fold (a total of 1000 times). This bacterial solution was used to inoculate the sub-plate. 100 µL of the bacterial solution was inoculated into each well of the sub-plate except the row A wells, and the row A wells were only added with 100 µL 7H9 medium with no bacterial solution added. The final concentrations of the drug tested were 64, 32, 16, 8, 4, 2, 1, 0.5, 0.25, 0.125, 0.0625 and 0 µg/mL respectively. The test sub-plate was placed in a 37°C incubator and the humidity was maintained at >80%.

After one week, 12.5 µL 7H9 medium containing 20% Tween 80 and 20 µL Alamar Blue (Invitrogen DAL1100) were added to the row A wells containing no bacteria and the row H wells containing bacteria. The plate was inoculated for 24 hours and observed. When the bacterial solution in the row H wells could reduce the added Alamar Blue to pink within 24 hours, Alamar Blue were added to all wells of the test plate and the plate was incubated at 37°C for 24 hours. Then the minimum inhibitory concentration (MIC) were determined.

The minimum inhibitory concentration (MIC) is defined as the minimum drug concentration that can completely inhibit the discoloration of Alamar Blue by visual observation, or the minimum drug concentration that can inhibit the formation of more than 90% reduced Alamar Blue as determined by a fluorometer.

The assay results were as shown in Table 7.

**Table 7 In vitro activity of the compound represented by formula (I) against M. bovis strains and M. tuberculosis H37Rv strains**

| Compound | *M. bovis* ATCC35737 (µg/mL) | *M. tuberculosis* H37Rv MABA (MIC) (µg/mL) | *M. tuberculosis* H37R LORA (MIC) (µg/mL) | Vero Cell (IC₅₀) (µg/mL) |
|---|---|---|---|---|
| (I) | <1 | <1 | 1 to 32 | >32 |

### Conclusion:

The compound represented by formula (I) exhibited a good inhibitory activity against both *M. bovis* BCG strain and *M. tuberculosis* H37Rv strain without cytotoxicity.

### Kinetic solubility and evaluation of bidirectional permeability in MDR1-MDCK cells

Test method for kinetic solubility:

Quantitative sample of the crystal form C of the compound represented by formula (II) was weighed and dissolved in pure DMSO to a final concentration of 10 mM. The test compound and the control compound (10 mM DMSO mother liquor, 10 µL per well) were added to a 96-well plate containing 490 µL buffer per well. After 2 minutes of vortexing, the sample plate were incubated for 24 hours at room temperature (22 ± 2°C) on a shaker. 200 µL sample was then transferred to a MultiScreen filter plate (polycarbonate membrane), filtered through a millipore vacuum manifold and the filtrate was collected. The concentration of the compound in the filtrate was determined by HPLC-UV Three UV standard solution of different concentrations and the solubility test sample were injected successively. Each sample was inserted twice, and the concentration was calculated by standard curve line and averaged.

### Test method for evaluation of bidirectional permeability in MDR1-MDCK cells

MDR1-MDCK cells permanently expressing human P-glycoprotein were inoculated in a 96-well Insert cell plate and cultured for 4-7 days to form convergent monolayer cells. The quality of the monolayer cells was verified by evaluation of unidirectional (A→B) permeability with fenoterol (low permeability marker) and propranolol (high permeability marker), and bidirectional permeability with Digoxin (a P-glycoprotein substrate). Each of the three compounds set two duplicate wells as control.

After the incubation, the sample solutions in the donor wells and receiver wells were immediately mixed with cold acetonitrile solution containing an internal standard. The amount of the intracellular accumulated compound was measured by lysing the cells with the cold acetonitrile solution containing an internal standard. The concentration of the test compound in all samples (including the starting dosing solution, the supernatant in the donor wells, the receiving solution, and the cell lysate) was analyzed by LC/MS/MS method. The concentration of the test compound was expressed by the ratio of the peak area of the compound to the peak area of the internal standard.

The standard conditions for the transport experiment of the compound represented by formula (I) were as follows:
Test concentration: 2 µM (DMSO≤1%);
Repeat: n=3;
Direction: bidirectional transport, including A→B and B→A these two directions;
Inoculated time: single time, 2.5 hours;
Buffer for transport: HBSS, pH 7.4;
Inoculated condition: 37°C, 5% CO₂, 95% relative humidity.
The tested results: Table 8 listed the kinetic solubility test for the crystal form A of the compound represented by formula (II) and the results of the bidirectional permeability evaluation of MDR1-MDCK cells for the compound represented by formula (I).

**Table 8 the kinetic solubility test for the crystal form A of the compound represented by formula (II) and the results of the bidirectional permeability evaluationof MDR1-MDCK cells for the compound represented by formula (I).**

| Test compound | kinetic solubility | parameter of MDR1-MDCK monolayer cell bidirectional permeability | | |
|---|---|---|---|---|
| | pH 2.0 (µM) | A to B (×10⁻⁶ cm/s) | B to A (×10⁻⁶ cm/s) | Efflux Ratio |
| OPC-67683 | 10.5 | 0.09 | 0.17 | 1.9 |
| crystal form A of the compound represented by formula (II)/ the compound represented by formula (I) | 32.16 | 20.92 | 15.85 | 0.76 |

### Conclusion:

The crystal form A of the compound represented by formula (II) is superior to OPC-67683 in kinetic solubility, which is beneficial to the absorption of drugs in the body and the study of preparations. Compared to the reference compound (OPC-67683), the compound represented by formula (I) has a better permeability, a significantly better absorption by the body, and can achieve a better anti-tuberculosis effect.

In vivo efficacy assay of the crystal form A of the compound represented by formula (II) in mouse model of chronic infection

### Design and method for assay:

The experimental principle was that aerosol inhalation infection of mice was carried out by using an atomizer to produce an aerosol with *Mycobacterium tuberculosis.* The infected mice were fed under normal conditions for 31 days to form a chronic lung infection of *Mycobacterium tuberculosis,* and then the mice were treated by orally administering drug. At the end of the experiment, the amount of the bacteria in the lungs of the mice was counted to evaluate the bactericidal efficacy of the drug.

The strain used in the experiment was *Mycobacterium tuberculosis* Erdman, ATCC 35801. The expansion medium was a broth medium based on Middlebrook 7H9, and a final concentration of 0.2% glycerol, 0.05% Tween 80, and 10% OADC (oleic acid-albumin-dextrose-catalase) were added. The bacteria were inoculated into the expansion medium and cultured at 37°C for 1 to 2 weeks to reach the logarithmic growth phase. Thereafter, the bacterial solution was collected by centrifugation at 3150 g at 4°C for 15 minutes. The collected *Mycobacterium tuberculosis* was washed twice with PBS containing 0.05% Tween 80 at 4°C. The bacterial solution was filtered at 4°C with a 8 µm filter to remove excessively large bacterial lumps. The bacterial solution was dispensed in 0.5 mL and stored in a -80°C ultra-low temperature freezer. The actual concentration of the prepared bacterial solution was determined by counting the colonies on a 7H11 plate.

Animals were adapted for feeding for at least two days in an animal feeding facility. On the onset day of the animal infection experiment, the bacterial solution prepared by the previous step was thawed and diluted to OD600 = 0.1 with Middlebrook 7H9, where the tuberculosis Bacillus bacterium was approximately 0.5-1 × 106 CFU/mL. 100 µL of the bacterial solution was plated on a 7H11 plate to determine the actual CFU concentration of the bacterial solution, and the bacterial solution was used as the inoculum.

10 mL of the bacterial inoculum was used to infect the animals through aerosol inhalation with a Middlebrook inhalation exposure system (IES) (Glas-Col, Terre Haure, IN, USA). Before infection, the IES was pre-heated for 15 minutes, and the mice were fixed. The bacteria solution was added to the nebulizer, and the aminals were exposed to the aerosol for inhalation for 1.5 hours. The IES was thoroughly sterilized immediately and the time of infection was recorded.

The mice in T4 group and T32 group were euthanized by CO₂ on the 4^{th} day and 32^{th} day respectively, and the CFU of *Mycobacterium tuberculosis* in the whole lung was counted. After the mice were euthanized by CO₂, the lungs were placed in 3 mL HBSS (Hanks Balanced Salt Solution) buffer and homogenized for 20-30 seconds, followed by sonication for 15 seconds. The homogenate was 10-fold diluted with the same HBSS buffer to make 1:10 to 1:10000 dilutions. A 7H11 plate was prepared using a 6-well plate, and 50 µL homogenate was inoculated per well. CFU was counted after all 6-well plates were cultured at 37°C for 18 days.

The vehicle control group and the treatment group were administered from the 32^{th} day to the 59th day after infection. One day later, the experiment was completed on the 60^{th} day, when the mice were euthanized by CO₂ and the CFU of *Mycobacterium tuberculosis* in the whole lung was counted.

The assay results were as shown in Table 9.

**Table 9**

| Group | Drug name | Dose (mg/kg) | Site | Number of colony | deviation | Log value reduced |
|---|---|---|---|---|---|---|
| T4 | / | 0 | T4 | 3.2E+02 | 1.3E+02 | - |
| T32 | / | 0 | T32 | 2.3E+06 | 1.1E+06 | - |
| T60, CMC | 0.5%CMC/0.5% Tween-80 | 0 | T60 CMC | 2.0E+06 | 1.0E+06 | - |
| RMP | Rifampin | 15 | RMP | 1.2E+04 | 3.6E+03 | 2.2 |
| A1 | the compound represented by formula (II) | 10 | A 10 | 2.7E+05 | 1.0E+05 | 0.9 |
| A2 | | 30 | A30 | 1.0E+05 | 5.1E+04 | 1.3 |
| A3 | | 100 | A 100 | 4.1E+04 | 2.0E+04 | 1.7 |
| B | the compound represented by formula (II) combined with pyrazinamide | A2 (30) combined with C (150) | A 30+C | 8.1E+04 | 8.8E+04 | 1.4 |
| C | pyrazinamide (PZA) | 150 | C | 1.2E+05 | 7.6E+04 | 1.2 |
| E | OPC-67683 | 100 | E | 2.3E+05 | 6.8E+04 | 0.9 |

Conclusion: The crystal form A of the compound represented by formula (II) exhibited an obvious dose-dependent in the *in vivo* pharmacodynamic model at 10, 30, 100 mg/kg. The CFU Log value was reduced by 1.7 at the dose of 100 mg/kg, which was significantly better than 0.9 Log exhibited by the reference compound OPC-67683. The CFU Log value reduced at 10mg/Kg was equivalent to the reference compound OPC-67683 at the dose of 100 mg/kg.

## Claims

1. A compound represented by formula (II)

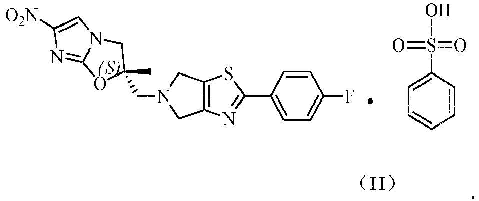

2. A crystal form A of the compound represented by formula (II), wherein the X-ray powder diffraction pattern of the crystal form A has characteristic diffraction peaks at the following 2θ angles: 5.74±0.2°, 13.29±0.2°, 17.79±0.2°, 18.48±0.2°, 20.25±0.2°, 20.51±0.2°, 22.07±0.2°, 23.33±0.2°.

3. The crystal form A of the compound represented by formula (II) as defined in claim 2, wherein the X-ray powder diffraction pattern is as shown in Figure 1.

4. A method for preparing the crystal form A as defined in claims 2-3, which comprises adding a compound represented by formula (I) in any form and benzenesulfonic acid to a solvent and crystallizing, 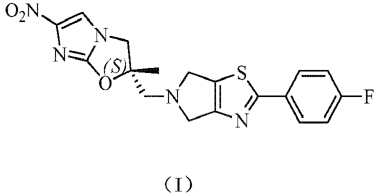 wherein,
the molar ratio of benzenesulfonic acid to the compound represented by formula (I) is from 1.2:1 to 1.0:1;
the solvent is 150 to 300 times the weight of the compound represented by formula (I);
the solvent is acetone.

5. A method for preparing the crystal form A as defined in claims 2-3, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is 50 to 150 times the weight of the compound represented by formula (I);
the solvent is butanone.

6. A method for preparing the crystal form A as defined in claims 2-3, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is 25 to 50 times the weight of the compound represented by formula (I);
the solvent is a mixed solvent of tetrahydrofuran and dimethyl sulfoxide.

7. The method for preparing the crystal form A as defined in claim 6, wherein, the volume ratio of tetrahydrofuran to dimethyl sulfoxide is from 6:1 to 10:1.

8. A method for preparing the crystal form A as defined in claims 2-3, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is 25 to 50 times the weight of the compound represented by formula (I);
the solvent is a mixed solvent of acetone and dimethyl sulfoxide.

9. The method for preparing the crystal form A as defined in claim 8, wherein the volume ratio of acetone to dimethyl sulfoxide is from 6:1 to 10:1.

10. A method for preparing the crystal form A as defined in claims 2-3, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is 10 to 20 times the weight of the compound represented by formula (I);
the solvent is a mixed solvent of acetone and acetic acid.

11. The method for preparing the crystal form A as defined in claim 10, wherein, the volume ratio of acetone to acetic acid is from 1:1 to 1.5:1.

12. A crystal form B of the compound represented by formula (II), wherein the X-ray powder diffraction pattern of the crystal form B has characteristic diffraction peaks at the following 2θ angles: 5.26±0.2°, 10.39±0.2°, 12.82±0.2°, 20.75±0.2°, 22.08±0.2°, 23.19±0.2°, 27.09±0.2°, 37.45±0.2°.

13. The crystal form B of the compound represented by formula (II) as defined in claim 12, wherein the X-ray powder diffraction pattern is as shown in Figure 4.

14. A method for preparing the crystal form B as defined in claim 12, which comprises adding a compound represented by formula (I) in any form and benzenesulfonic acid to a solvent and crystallizing, wherein,
the molar ratio of benzenesulfonic acid to the compound represented by formula (I) is from 1.2:1 to 1.0:1;
the solvent is 150 to 300 times the weight of the compound represented by formula (I);
the solvent is acetone.

15. A compound represented by formula (III)

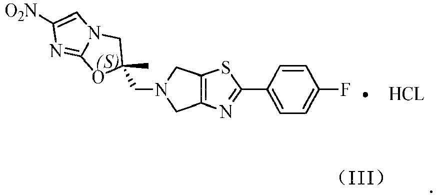

16. A compound represented by formula (IV)

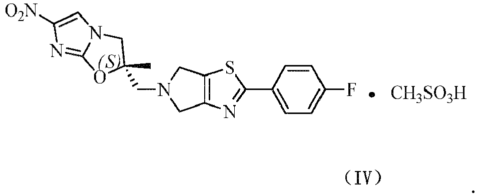

17. A compound represented by formula (V)

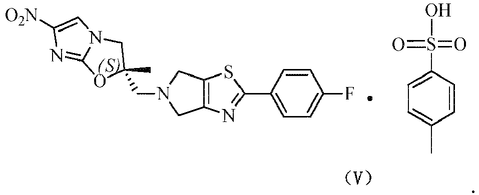

18. A crystal form C of the compound represented by formula (I), wherein the X-ray powder diffraction pattern of the crystal form C has characteristic diffraction peaks at the following 2θ angles: 7.18±0.2°, 10.78±0.2°, 14.10±0.2°, 14.41±0.2°, 15.36±0.2°, 23.72±0.2°, 25.36±0.2°, 27.49±0.2°.

19. The crystal form C of the compound represented by formula (I) as defined in claim 18, wherein the X-ray powder diffraction pattern is as shown in Figure 6.

20. A use of the compound as defined in claim 1 or the crystal form A as defined in claims 2-3 or the crystal form B as defined in claims 12-13 in manufacturing a medicament for preventing and treating an infection caused by *Mycobacterium tuberculosis* or other microbes.

21. A use of the compound as defined in claims 15-17 in manufacturing a medicament for preventing and treating an infection caused by *Mycobacterium tuberculosis* or other microbes.

22. A use of the crystal form C as defined in claim 18 in manufacturing a medicament for preventing and treating an infection caused by *Mycobacterium tuberculosis* or other microbes.
